# EUROPEAN PATENT APPLICATION

(11) **EP 2 765 141 A1**
(43) Date of publication of application: **13.08.2014**
(21) Application number: 14159277.4
(22) Date of filing: 27.09.2012
(51) Int. Cl.: C07K 16/28, C07K 16/30, C12N 15/113, C12Q 1/68, A61K 31/713

(54) **Compositions comprising agents that inhibit neuroplilin and tolloid like 2**

(30) Priority: 28.09.2011 GB 201116702
(62) Divisional of application: 12780520.8
(71) Applicant: The University of York, York, Yorkshire YO10 5DD (GB)
(72) Inventor: Birnie, Richard, York Yorkshire YO30 6EA (GB); Maitland, Norman, York Yorkshire YO10 5DD (GB)
(74) Representative: Docherty, Robert Charles

(57) **Abstract**

We disclose agents that inhibit the expression of NETO-2 which has elevated expression in cancer stem cells; the use of NETO- 2 as a diagnostic or prognostic marker of tumour initiation; the use NETO-2 polypeptides in the identification of agents that inhibit activity; and including antibodies that bind NETO-2 and vaccines comprising NETO-2 polypeptides.

## Description

### Field of the Invention

The disclosure relates to agents that inhibit the expression or activity of neuropilin and tolloid like 2 [NETO-2] which has elevated expression in cancer stem cells; the monitoring of expression of NETO-2 as a diagnostic or prognostic marker of tumour initiation; the use NETO-2 polypeptide in the identification of agents that inhibit activity; and including vaccines comprising NETO-2 polypeptides and antibodies that binds NETO-2.

### Background to the Invention

The term "stem cell" represents a generic group of undifferentiated cells that possess the capacity for self-renewal while retaining varying potential to form differentiated cells and tissues. Stem cells can be pluripotent or multipotent. A pluripotent stem cell is a cell that has the ability to form all tissues found in an intact organism although the pluripotent stem cell cannot form an intact organism. A multipotent cell has a restricted ability to form differentiated cells and tissues. Typically adult stem cells are multipotent stem cells and are the precursor stem cells or lineage restricted stem cells that have the ability to form some cells or tissues and replenish senescing or damaged cells/tissues. Generally they cannot form all tissues found in an organism although some reports have claimed a greater potential for such 'adult' stem cells than originally thought.

Evidence suggests that tumours are clonal and are therefore derived from a single cell. However, there are few studies that identify and characterize those cells types that are responsible for maintaining tumour cell growth. Some have searched for these so called "cancer stem cells". The concept of a cancer stem cell within a more differentiated tumour mass, as an aberrant form of normal differentiation, is now gaining acceptance over the current model of oncogenesis in which all tumour cells are equivalent both in growth and tumour-initiating capacity [Hamburger AW, Salmon SE: Primary bioassay of human tumor stem cells. Science 1977, 197: 461463; Pardal R, Clarke MF, Morrison SJ: Applying the principles of stem cell biology to cancer. Nat. Rev. Cancer 2003, 3: 895902.] For example, in leukaemia, the ability to initiate new tumour growth resides in a rare phenotypically distinct subset of tumour cells [Bonnet D, Dick J.E. Human acute myeloid leukaemia is organized as a hierarchy that originates from a primitive hematopoietic cell Nat. Med. 1997, 3: 730737] which are defined by the expression of CD34 and CD38 surface antigens and have been termed leukaemia stem cells.

Similar tumour-initiating cells have also been found in 'solid' cancers such as prostate [Collins AT, Berry PA, Hyde C, Stower MJ, Maitland NJ: Prospective Identification of Tumorigenic Prostate Cancer Stem Cells. Cancer Res. 2005, 65: 1094610951], breast [Al Hajj M, Wicha MS, BenitoHernandez A, Morrison SJ, Clarke MF: Prospective identification of tumorigenic breast cancer cells. Proc Natl Acad Sci U S A 2003, 100: 39833988], brain [Singh SK, Hawkins C, Clarke ID, Squire JA, Bayani J, Hide T, Henkelman RM, Cusimano MD, Dirks PB: Identification of human brain tumour initiating cells. Nature 2004, 432: 396401], lung [Kim CF, Jackson EL, Woolfenden AE, Lawrence S, Babar I., Vogel S, Crowley D, Bronson RT, Jacks T: Identification of bronchioalveolar stem cells in normal lung and lung cancer. Cell 2005, 121: 823-835] colon [O'Brien CA, Pollett A, Gallinger S, Dick JE: A human colon cancer cell capable of initiating tumour growth in immunodeficient mice. Nature 2007, 445: 106110; RicciVitiani L, Lombardi DG, Pilozzi E, Biffoni M, Todaro M, Peschle C, De Maria R: Identification and expansion of human colon cancer initiating cells. Nature 2007, 445: 111115]; and gastric cancers [Houghton J, Stoicov C, Nomura S, Rogers AB, Carlson J, Li H, Cai X, Fox JG, Goldenring JR, Wang TC: Gastric cancer originating from bone marrow derived cells. Science 2004, 306: 15681571].

This disclosure relates to the identification of NETO-2 which has enhanced expression in cancer stem cells and in particular prostate cancer stem cells and wherein expression is correlated with tumour cell initiation. In WO2005/089043 we describe the isolation of prostate stem cells which have been directly isolated from lymph node and prostate glands from a series of patient samples. These stem cells express markers that characterise the cells with stem cell properties. The following markers are typically expressed as prostate stem cell markers; human epithelial antigen (HEA), CD44, α₂β₁^{hi} and CD133. This disclosure identifies NETO-2 the expression of which is critical for colony formation which is the in initiating step in the formation of a tumour. We disclose that inhibition of expression of NETO-2 results in a failure to form colonies and a subsequent failure to form a tumour *in vivo.* The expression of NETO-2 therefore represents the early stages of tumour formation and allows an early therapeutic intervention with consequent inhibition of tumour formation. The detection of NETO-2 also serves as a diagnostic or prognostic marker of the early stages of tumour formation. NETO-2 exists as two isoforms. The full length isoform is 525 amino acids in length. A second isoform is a shorter version and is missing amino acid residues 1-324 and has a different amino acid sequence between amino acid residues 325-333. NETO-2 is a single span transmembrane receptor and is known interact with glutamate receptor which is primarily expressed in the brain.

### Statements of Invention

According to an aspect of the invention there is provided an agent that inhibits the expression of NETO-2 or the activity of NETO-2 wherein said expression/activity is enhanced in a cancer stem cell and wherein the agent inhibits tumour initiation.

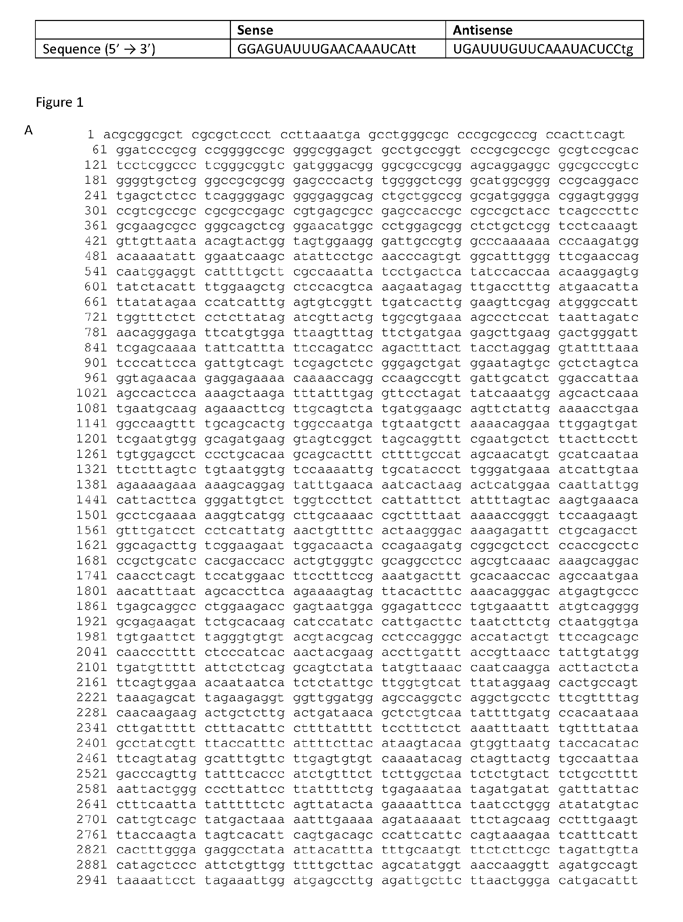

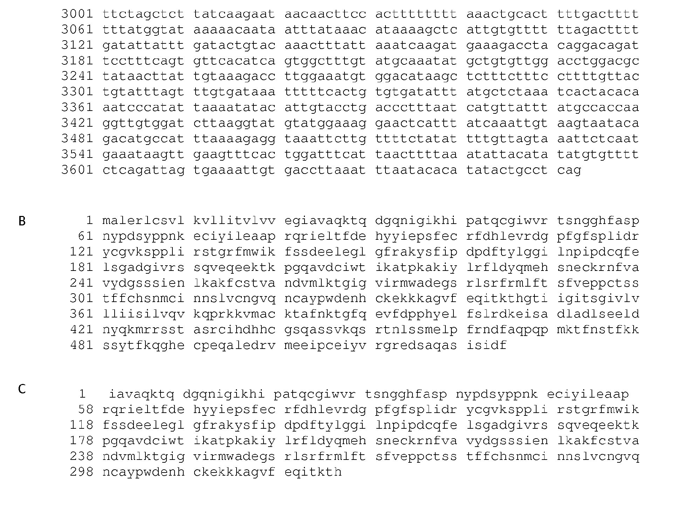

Preferably NETO-2 comprises or consists of the nucleotide sequence as represented in Figure 1 a.

In a preferred embodiment of the invention said agent is an antisense oligonucleotide or RNA.

According to a further aspect of the invention there is provided a composition comprising one or more antisense oligonucleotide or RNA molecules wherein said oligonucleotide or antisense RNA molecule comprise a nucleotide sequence adapted to anneal to a sense nucleotide sequence derived from at least one gene represented by the sense sequence presented in Figure 1a.

In a preferred embodiment of the invention said composition is a pharmaceutical composition.

In a preferred embodiment of the invention said composition comprises, one, two, three or four antisense oligonucleotides or RNA molecules.

In a preferred embodiment of the invention said composition consists essentially of one or more oligonucleotides or antisense RNA molecules and physiologically compatible excipients and/or adjuvants.

In a preferred embodiment of the invention said antisense RNA molecule is part of a siRNA or shRNA molecule.

A technique to specifically ablate gene function is through the introduction of double stranded RNA, also referred to as small inhibitory or interfering RNA (siRNA), into a cell which results in the destruction of mRNA complementary to the sequence included in the siRNA molecule. The siRNA molecule comprises two complementary strands of RNA (a sense strand and an antisense strand) annealed to each other to form a double stranded RNA molecule. The siRNA molecule is typically derived from exons of the gene which is to be ablated. The mechanism of RNA interference is being elucidated. Many organisms respond to the presence of double stranded RNA by activating a cascade that leads to the formation of siRNA. The presence of double stranded RNA activates a protein complex comprising RNase III which processes the double stranded RNA into smaller fragments (siRNAs, approximately 21-29 nucleotides in length) which become part of a ribonucleoprotein complex. The siRNA acts as a guide for the RNase complex to cleave mRNA complementary to the antisense strand of the siRNA thereby resulting in destruction of the mRNA.

In a preferred embodiment of the invention said antisense RNA molecule is between 19 nucleotides [nt] and 29nt in length. More preferably still said antisense RNA molecule is between 21 nt and 27nt in length. Preferably said antisense RNA molecule is about 21 nt in length.

In a preferred embodiment of the invention said antisense RNA consists of 21 nt.

In an alternative preferred embodiment of the invention said siRNA is represented by the nucleotide sequences presented in Table 1.

In a preferred embodiment of the invention said antisense, siRNA or shRNA includes modified nucleotides.

The term "modified" as used herein describes a nucleic acid molecule in which;
i) at least two of its nucleotides are covalently linked via a synthetic internucleoside linkage (i.e., a linkage other than a phosphodiester linkage between the 5' end of one nucleotide and the 3' end of another nucleotide). Alternatively or preferably said linkage may be the 5' end of one nucleotide linked to the 5' end of another nucleotide or the 3' end of one nucleotide with the 3' end of another nucleotide; and/or
ii) a chemical group, such as cholesterol, not normally associated with nucleic acids has been covalently attached to the double stranded nucleic acid.
iii) Preferred synthetic internucleoside linkages are phosphorothioates, alkylphosphonates, phosphorodithioates, phosphate esters, alkylphosphonothioates, phosphoramidates, carbamates, phosphate triesters, acetamidates, peptides, and carboxymethyl esters.

The term "modified" also encompasses nucleotides with a covalently modified base and/or sugar. For example, modified nucleotides include nucleotides having sugars which are covalently attached to low molecular weight organic groups other than a hydroxyl group at the 3' position and other than a phosphate group at the 5' position. Thus modified nucleotides may also include 2' substituted sugars such as 2'-O-methyl-; 2-O-alkyl; 2-O-allyl; 2'-S-alkyl; 2'-S-allyl; 2'- fluoro-; 2'-halo or 2;azido-ribose, carbocyclic sugar analogues α-anomeric sugars; epimeric sugars such as arabinose, xyloses or lyxoses, pyranose sugars, furanose sugars, and sedoheptulose.

Modified nucleotides are known in the art and include, by example and not by way of limitation, alkylated purines and/or pyrimidines; acylated purines and/or pyrimidines; or other heterocycles. These classes of pyrimidines and purines are known in the art and include, pseudoisocytosine; N4, N4-ethanocytosine; 8-hydroxy-N6-methyladenine; 4-acetylcytosine, 5-(carboxyhydroxylmethyl) uracil; 5-fluorouracil; 5-bromouracil;5-carboxymethylaminomethyl-2-thiouracil; 5 carboxymethylaminomethyl uracil; dihydrouracil; inosine; N6-isopentyl-adenine; I-methyladenine; 1-methylpseudouracil; 1-methylguanine; 2,2-dimethylguanine; 2-methyladenine; 2-methylguanine; 3-methylcytosine; 5-methylcytosine; N6-methyladenine; 7-methylguanine; 5-methylaminomethyl uracil; 5-methoxy amino methyl-2-thiouracil; β-D-mannosylqueosine; 5-methoxycarbonylmethyluracil; 5-methoxyuracil; 2 methylthio-N6-isopentenyladenine; uracil-5-oxyacetic acid methyl ester; psueouracil; 2-thiocytosine; 5-methyl-2 thiouracil, 2-thiouracil; 4-thiouracil; 5-methyluracil; N-uracil-5-oxyacetic acid methylester; uracil 5-oxyacetic acid; queosine; 2-thiocytosine; 5-propyluracil; 5-propylcytosine; 5-ethyluracil; 5-ethylcytosine; 5-butyluracil; 5-pentyluracil; 5-pentylcytosine; and 2,6,-diaminopurine; methylpsuedouracil; 1-methylguanine; 1-methylcytosine. Modified double stranded nucleic acids also can include base analogs such as C-5 propyne modified bases (see Wagner et al., Nature Biotechnology 14:840-844, 1996).

In an alternative preferred embodiment of the invention said pharmaceutical composition includes a carrier adapted to deliver said antisense RNA to a cell or tissue.

The delivery of antisense oligonucleotide, siRNA or shRNA is achieved using delivery vehicles known in the art. For example siRNA can be chemically modified and conjugated to a lipophilic cholesterol moiety at the 3' end of the sense strand. Cationic delivery systems can also be employed in the delivery of siRNA. These include cationic lipids and liposomes, cationic polymers, cationic dendrimers and cationic cell penetrating peptides. The cationic delivery vehicles have a common positive charge which facilitates complex formation with negatively charged siRNA. Commercially available examples of liposome based delivery vehicles include Lipofectin, RNAifect, Oligofectamine, Lipofectamine and TransIT TKO have been used in vitro. DOTAP (N [1-(2, 3-dioleoyloxy)]-N, N, N-trimethyl ammonium propane) and Oligfectamine have been utilised *in vivo.* Other liposome based delivery vehicle includes solid nucleic acid lipid particles [SNALPs] which are also conjugated with polyethylene glycol. Peptide delivery vehicles have also been successful in delivering siRNA. Pegylated polyethyleneimine [PEI] comprising RGD peptides have been used to target siRNA to angiogenesis factors such as VEGF. Atelocollagen has been used in the delivery of siRNA to tumours *in vivo.* Delivery of siRNA has also been demonstrated using cyclodextrin polymers. A yet further example of a siRNA delivery vehicle are self assembled LPD nanoparticles which have been used to deliver to solid and metastatic tumours. LPD nanoparticles comprise cationic lipids combined with protamine which interacts with negatively charged siRNA. Pegylated versions of LPD nanoparticles are also known which have improved pharmacokinetics.

In a preferred embodiment of the invention said antibody is a polyclonal antibody.

In an alternative preferred embodiment of the invention said antibody is a monoclonal antibody.

Antibodies, also known as immunoglobulins, are protein molecules which have specificity for foreign molecules (antigens). Immunoglobulins (Ig) are a class of structurally related proteins consisting of two pairs of polypeptide chains, one pair of light (L) (low molecular weight) chain (κ or λ), and one pair of heavy (H) chains (γ, α, µ, δ and ε), all four linked together by disulphide bonds. Both H and L chains have regions that contribute to the binding of antigen and that are highly variable from one Ig molecule to another. In addition, H and L chains contain regions that are non-variable or constant. The L chains consist of two domains. The carboxy-terminal domain is essentially identical among L chains of a given type and is referred to as the "constant" (C) region. The amino terminal domain varies from L chain to L chain and contributes to the binding site of the antibody. Because of its variability, it is referred to as the "variable" (V) region. The H chains of Ig molecules are of several classes, α, µ, σ, α, and γ (of which there are several sub-classes). An assembled Ig molecule consisting of one or more units of two identical H and L chains, derives its name from the H chain that it possesses. Thus, there are five Ig isotypes: IgA, IgM, IgD, IgE and IgG (with four sub-classes based on the differences in the H chains, i.e., IgG1, IgG2, IgG3 and IgG4). Further detail regarding antibody structure and their various functions can be found in, Using Antibodies: A laboratory manual, Cold Spring Harbour Laboratory Press.

In a preferred embodiment of the invention said fragment is a single chain antibody fragment.

Various fragments of antibodies are known in the art, e.g. Fab, Fab₂, F(ab')₂, Fv, Fc, Fd" etc. A Fab fragment is a multimeric protein consisting of the immunologically active portions of an immunoglobulin heavy chain variable region and an immunoglobulin light chain variable region, covalently coupled together and capable of specifically binding to an antigen. Fab fragments are generated via proteolytic cleavage (with, for example, papain) of an intact immunoglobulin molecule. A Fab₂ fragment comprises two joined Fab fragments. When these two fragments are joined by the immunoglobulin hinge region, a F(ab')₂ fragment results. An Fv fragment is multimeric protein consisting of the immunologically active portions of an immunoglobulin heavy chain variable region and an immunoglobulin light chain variable region covalently coupled together and capable of specifically binding to an antigen. A fragment could also be a single chain polypeptide containing only one light chain variable region, or a fragment thereof that contains the three CDRs of the light chain variable region, without an associated heavy chain variable region, or a fragment thereof containing the three CDRs of the heavy chain variable region, without an associated light chain moiety; and multi specific antibodies formed from antibody fragments, this has for example been described in US patent No 6,248,516. Fv fragments or single region (domain) fragments are typically generated by expression in host cell lines of the relevant identified regions. These and other immunoglobulin or antibody fragments are within the scope of the invention and are described in standard immunology textbooks such as Paul, Fundamental Immunology or Janeway's Immunobiology, Murphy, K., Travers, P. & Walport P.

Molecular biology now allows direct synthesis (via expression in cells or chemically) of these fragments, as well as synthesis of combinations thereof. A fragment of an antibody or immunoglobulin can also have bispecific function as described above.

In a preferred embodiment of the invention said antibody is a chimeric antibody.

In an alternative preferred embodiment of the invention said antibody is a humanized or human antibody.

Chimeric antibodies are recombinant antibodies in which all of the V-regions of a mouse or rat antibody are combined with human antibody C-regions. Humanised antibodies are recombinant hybrid antibodies which fuse the complementarity determining regions from a rodent antibody V-region with the framework regions from the human antibody V-regions. The C-regions from the human antibody are also used. The complementarity determining regions (CDRs) are the regions within the N-terminal domain of both the heavy and light chain of the antibody to where the majority of the variation of the V-region is restricted. These regions form loops at the surface of the antibody molecule. These loops provide the binding surface between the antibody and antigen.

Antibodies from non-human animals provoke an immune response to the foreign antibody and its removal from the circulation. Both chimeric and humanised antibodies have reduced antigenicity when injected to a human subject because there is a reduced amount of rodent (i.e. foreign) antibody within the recombinant hybrid antibody, while the human antibody regions do not ellicit an immune response. This results in a weaker immune response and a decrease in the clearance of the antibody. This is clearly desirable when using therapeutic antibodies in the treatment of human diseases. Humanised antibodies are designed to have less "foreign" antibody regions and are therefore thought to be less immunogenic than chimeric antibodies.

Reviews of current delivery vehicles can be found in Molecular Pharmaceutics 2008 Vol 6[3] p651-658; The AAPS Journal 2009 Vol 11 [4] p639; Pharmaceutical Research 2009, Vol 26[3] p657; and Nature Reviews 2009 Vol 8, p129.

When administered the compositions of the present invention are administered in pharmaceutically acceptable preparations. Such preparations may routinely contain pharmaceutically acceptable concentrations of salt, buffering agents, preservatives, compatible carriers and supplementary anti-cancer agents.

The compositions of the invention can be administered by any conventional route, including injection or by gradual infusion over time. Treatment may be topical or systemic. The administration may, for example, be oral, intravenous, intraperitoneal, intramuscular, intracavity, subcutaneous, transdermal, transepithelial or intra bone marrow administration or by direct injection into the tumour mass.

The compositions of the invention are administered in effective amounts. An "effective amount" is that amount of a composition that alone, or together with further doses, produces the desired response. In the case of treating a particular disease, such as cancer, the desired response is inhibiting the progression of the disease. This may involve only slowing the progression of the disease temporarily, although more preferably, it involves halting the progression of the disease permanently. This can be monitored by routine methods.

Such amounts will depend, of course, on the particular condition being treated, the severity of the condition, the individual patient parameters including age, physical condition, size and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation. It is generally preferred that a maximum dose of the individual components or combinations thereof be used, that is, the highest safe dose according to sound medical judgment. It will be understood by those of ordinary skill in the art, however, that a patient may insist upon a lower dose or tolerable dose for medical reasons, psychological reasons or for virtually any other reasons.

The pharmaceutical compositions used in the foregoing methods preferably are sterile and contain an effective amount of an agent according to the invention for producing the desired response in a unit of weight or volume suitable for administration to a patient.

The doses of the antisense RNA according to the invention administered to a subject can be chosen in accordance with different parameters, in particular in accordance with the mode of administration used and the state of the subject. Other factors include the desired period of treatment. In the event that a response in a subject is insufficient at the initial doses applied, higher doses (or effectively higher doses by a different, more localized delivery route) may be employed to the extent that patient tolerance permits.

In general, doses of antisense RNA [e.g., siRNA] of between 1nM - 1µM generally will be formulated and administered according to standard procedures. Preferably doses can range from 1nM-500nM, 5nM-200nM, and 10nM-100nM. Other protocols for the administration of compositions will be known to one of ordinary skill in the art, in which the dose amount, schedule of injections, sites of injections, mode of administration and the like vary from the foregoing. The administration of compositions to mammals other than humans, (e.g. for testing purposes or veterinary therapeutic purposes), is carried out under substantially the same conditions as described above. A subject, as used herein, is a mammal, preferably a human, and including a non-human primate, cow, horse, pig, sheep, goat, dog, cat or rodent.

When administered, the pharmaceutical preparations of the invention are applied in pharmaceutically-acceptable amounts and in pharmaceutically-acceptable compositions. The term "pharmaceutically acceptable" means a non-toxic material that does not interfere with the effectiveness of the biological activity of the active ingredients. Such preparations may routinely contain salts, buffering agents, preservatives, compatible carriers, and optionally other therapeutic agents' (e.g. anti-inflammatory agents such as steroids, non-steroidal anti-inflammatory agents, chemotherapeutic agents). When used in medicine, the salts should be pharmaceutically acceptable, but non-pharmaceutically acceptable salts may conveniently be used to prepare pharmaceutically-acceptable salts thereof and are not excluded from the scope of the invention. Such pharmacologically and pharmaceutically-acceptable salts include, but are not limited to, those prepared from the following acids: hydrochloric, hydrobromic, sulfuric, nitric, phosphoric, maleic, acetic, salicylic, citric, formic, malonic, succinic, and the like. Also, pharmaceutically-acceptable salts can be prepared as alkaline metal or alkaline earth salts, such as sodium, potassium or calcium salts.

Compositions may be combined, if desired, with a pharmaceutically-acceptable carrier. The term "pharmaceutically-acceptable carrier" as used herein means one or more compatible solid or liquid fillers, diluents or encapsulating substances which are suitable for administration into a human. The term "carrier" in this context denotes an organic or inorganic ingredient, natural or synthetic, with which the active ingredient is combined to facilitate the application, (e.g. liposome or immuno-liposome). The components of the pharmaceutical compositions also are capable of being co-mingled with the molecules of the present invention, and with each other, in a manner such that there is no interaction which would substantially impair the desired pharmaceutical efficacy.

The pharmaceutical compositions may contain suitable buffering agents, including: acetic acid in a salt; citric acid in a salt; boric acid in a salt; and phosphoric acid in a salt. The pharmaceutical compositions also may contain, optionally, suitable preservatives, such as: benzalkonium chloride; chlorobutanol; parabens and thimerosal.

The pharmaceutical compositions may conveniently be presented in unit dosage form and may be prepared by any of the methods well-known in the art of pharmacy. All methods include the step of bringing the active agent into association with a carrier which constitutes one or more accessory ingredients. In general, the compositions are prepared by uniformly and intimately bringing the active compound into association with a liquid carrier, a finely divided solid carrier, or both, and then, if necessary, shaping the product.

Compositions suitable for oral administration may be presented as discrete units, such as capsules, tablets, lozenges, each containing a predetermined amount of the active compound. Other compositions include suspensions in aqueous liquids or non-aqueous liquids such as syrup, elixir or an emulsion or as a gel. Compositions may be administered as aerosols and inhaled.

Compositions suitable for parenteral administration conveniently comprise a sterile aqueous or non-aqueous preparation of agent, which is preferably isotonic with the blood of the recipient. This preparation may be formulated according to known methods using suitable dispersing or wetting agents and suspending agents. The sterile injectable preparation also may be a sterile injectable solution or suspension in a non-toxic parenterally-acceptable diluent or solvent, for example, as a solution in 1, 3-butane diol. Among the acceptable solvents that may be employed are water, Ringer's solution, and isotonic sodium chloride solution. In addition, sterile, fixed oils are conventionally employed as a solvent or suspending medium. For this purpose any bland fixed oil may be employed including synthetic mono-or di-glycerides. In addition, fatty acids such as oleic acid may be used in the preparation of injectables. Carrier formulation suitable for oral, subcutaneous, intravenous, intramuscular, etc. administrations can be found in Remington's Pharmaceutical Sciences, Mack Publishing Co., Easton, PA.

In a preferred embodiment of the invention said composition includes an additional chemotherapeutic agent.

A general definition of "chemotherapeutic agent" is an agent that typical is a small chemical compound that kills cells in particular diseased cells, for example cancer cells. The agents can be divided with respect to their structure or mode of action. For example, chemotherapeutic agents include alkylating agents, anti-metabolites, anthracyclines, alkaloids, plant terpenoids and toposisomerase inhibitors. Chemotherapeutic agents typically produce their effects on cell division or DNA synthesis.

In a preferred embodiment of the invention said chemotherapeutic agent is an alkylating agent.

Preferably said alkylating agent is selected from the group consisting of: cisplatin, carboplatin or oxaliplatin.

In a preferred embodiment of the invention said chemotherapuetic agent is an anti-metabolic drug.

In a preferred embodiment of the invention said drug is a purine analogue. In an alternative preferred embodiment of the invention said drug is a pyrimidine analogue.

Purine analogues are known in the art; for example thioguanine is used to treat acute leukaemia; fludarabine inhibits the function of DNA polymerases, DNA primases and DNA ligases and is specific for cell-cycle S-phase; pentostatin and cladribine are adenosine analogues and are effective against hairy cell leukaemias. A further example is mecrcaptopurine which is an adenine analogue. Pyrimidine analogues are similarly known in the art. For example, 5-fluorouracil (5-FU), floxuridine and cytosine arabinoside. 5-FU has been used for many years in the treatment of breast, colorectal cancer, pancreatic and other cancers. 5-FU can also been formed from the pro-drug capecitabine which is converted to 5-FU in the tumour.

In a preferred embodiment of the invention said chemotherapeutic agent is 5-fluorouracil.

In a preferred embodiment of the invention said anti-metabolic drug is administered with leucovorin.

Leucovorin, also known as folinic acid, is administered as an adjuvant in cancer chemotherapy and which enhances the inhibitory effects of 5-FU on thymidylate synthase.

In a further preferred embodiment of the invention said chemotherapeutic agent is an alkaloid; preferably said alkaloid is a vinca alkaloid, for example vincristine or vinblastine.

In a yet further preferred embodiment of the invention said chemotherapeutic agent is a terpenoid; preferably a taxane e.g. palitaxel.

According to an aspect of the invention there is provided the use of a polypeptide encoded by a nucleic acid molecule comprising a nucleotide sequence as represented in Figure 1 a for the identification of agents that modulate the activity of said polypeptide.

According to an aspect of the invention there is provided a screening method for the identification of an agent that inhibits the activity of a cancer stem cell gene expression product comprising:
i) providing a polypeptide encoded by a nucleic acid molecule comprising a nucleotide sequence as represented in Figure 1a;
ii) providing at least one candidate agent to be tested;
iii) forming a preparation that is a combination of (i) and (ii) above; and
iv) testing the effect of said agent on the activity of said polypeptide.

According to a further aspect of the invention there is provided a modelling method to determine the association of an agent with a cancer stem cell gene expression product comprising:
i) providing computational means to perform a fitting operation between an agent and a polypeptide comprising or consisting of the amino acid sequence in Figure 1b or 1c; and
ii) analysing the results of said fitting operation to quantify the association between the agent and the polypeptide.

The rational design of binding entities for proteins is known in the art and there are a large number of computer programs that can be utilised in the modelling of 3-dimensional protein structures to determine the binding of chemical entities to functional regions of proteins and also to determine the effects of mutation on protein structure. This may be applied to binding entities and also to the binding sites for such entities. The computational design of proteins and/or protein ligands demands various computational analyses which are necessary to determine whether a molecule is sufficiently similar to the target protein or polypeptide. Such analyses may be carried out in current software applications, such as the Molecular Similarity application of QUANTA (Molecular Simulations Inc., Waltham, Mass.) version 3.3, and as described in the accompanying User's Guide, Volume 3 pages. 134-135. The Molecular Similarity application permits comparisons between different structures, different conformations of the same structure, and different parts of the same structure. Each structure is identified by a name. One structure is identified as the target (i.e., the fixed structure); all remaining structures are working structures (i.e. moving structures). When a rigid fitting method is used, the working structure is translated and rotated to obtain an optimum fit with the target structure.

The person skilled in the art may use one of several methods to screen chemical entities or fragments for their ability to associate with a target. The screening process may begin by visual inspection of the target on the computer screen, generated from a machine-readable storage medium. Selected fragments or chemical entities may then be positioned in a variety of orientations, or docked, within the binding pocket.

Useful programs to aid the person skilled in the art in connecting the individual chemical entities or fragments include: CAVEAT (P. A. Bartlett et al, "CAVEAT: A Program to Facilitate the Structure-Derived Design of Biologically Active Molecules". In Molecular Recognition in Chemical and Biological Problems", Special Pub., Royal Chem. Soc., 78, pp. 182-196 (1989)). CAVEAT is available from the University of California, Berkeley, California. 3D Database systems such as MACCS-3D (MDL Information Systems, San Leandro, California). This is reviewed in Y. C. Martin, "3D Database Searching in Drug Design", J. Med. Chem., 35, pp. 2145-2154 (1992); and HOOK (available from Molecular Simulations, Burlington, Mass.).

Once the agent has been optimally selected or designed, as described above, substitutions may then be made in some of its atoms or side groups in order to improve or modify its binding properties. Generally, initial substitutions are conservative, i.e., the replacement group will have approximately the same size, shape, hydrophobicity and charge as the original group. The computational analysis and design of molecules, as well as software and computer systems are described in US Patent No 5,978,740 which is included herein by reference.

According to a further aspect of the invention there is provided a vaccine composition comprising a polypeptide selected from the group consisting of:
i) a polypeptide encoded by a nucleotide sequence as represented in Figure 1a, or an antigenic fragment thereof;
ii) a polypeptide encoded by a nucleotide sequence wherein said sequence is degenerate as a result of the genetic code to the nucleotide sequence defined in (i);
iii) a polypeptide or antigenic fragment comprising an amino acid sequence wherein said sequence is modified by addition deletion or substitution of at least one amino acid residue as represented in Figures 1b or 1c, wherein said composition optionally includes an adjuvant and/or carrier.

In a preferred embodiment of the invention said antigenic fragment is an extracellular domain of said polypeptide.

In a preferred embodiment of the invention said extracellular domain comprises or consists of the amino acid sequence as represented in Figure 1 c.

In a preferred embodiment of the invention said composition includes an adjuvant and/or carrier.

In a preferred embodiment of the invention said adjuvant is selected from the group consisting of: cytokines selected from the group consisting of GMCSF, interferon gamma, interferon alpha, interferon beta, interleukin 12, interleukin 23, interleukin 17, interleukin 2, interleukin 1, TGF, TNFα, and TNFβ.

In a further alternative embodiment of the invention said adjuvant is a TLR agonist such as CpG oligonucleotides, flagellin, monophosphoryl lipid A, poly I:C and derivatives thereof.

In a preferred embodiment of the invention said adjuvant is a bacterial cell wall derivative such as muramyl dipeptide (MDP) and/or trehalose dicorynomycolate (TDM).

An adjuvant is a substance or procedure which augments specific immune responses to antigens by modulating the activity of immune cells. Examples of adjuvants include, by example only, Freunds adjuvant, muramyl dipeptides, liposomes. An adjuvant is therefore an immunomodulator. A carrier is an immunogenic molecule which, when bound to a second molecule augments immune responses to the latter. The term carrier is construed in the following manner. A carrier is an immunogenic molecule which, when bound to a second molecule augments immune responses to the latter. Some antigens are not intrinsically immunogenic yet may be capable of generating antibody responses when associated with a foreign protein molecule such as keyhole-limpet haemocyanin or tetanus toxoid. Such antigens contain B-cell epitopes, but no T cell epitopes. The protein moiety of such a conjugate (the "carrier" protein) provides T-cell epitopes which stimulate helper T-cells that in turn stimulate antigen-specific B-cells to differentiate into plasma cells and produce antibody against the antigen.

According to a further aspect of the invention there is provided a vaccine according to the invention for use in the treatment of cancer.

As used herein, the term "cancer" refers to cells having the capacity for autonomous growth, i.e., an abnormal state or condition characterized by rapidly proliferating cell growth. The term is meant to include all types of cancerous growths or oncogenic processes, metastatic tissues or malignantly transformed cells, tissues, or organs, irrespective of histopathologic type or stage of invasiveness. The term "cancer" includes malignancies of the various organ systems, such as those affecting, for example, lung, breast, thyroid, lymphoid, gastrointestinal, and genito-urinary tract, as well as adenocarcinomas which include malignancies such as most colon cancers, renal-cell carcinoma, prostate cancer and/or testicular tuours, non-small cell carcinoma of the lung, cancer of the small intestine and cancer of the esophagus. The term "carcinoma" is art recognized and refers to malignancies of epithelial or endocrine tissues including respiratory system carcinomas, gastrointestinal system carcinomas, genitourinary system carcinomas, testicular carcinomas, breast carcinomas, prostatic carcinomas, endocrine system carcinomas, and melanomas. Exemplary carcinomas include those forming from tissue of the cervix, lung, prostate, breast, head and neck, colon and ovary. The term "carcinoma" also includes carcinosarcomas, e.g., which include malignant tumours composed of carcinomatous and sarcomatous tissues. An "adenocarcinoma" refers to a carcinoma derived from glandular tissue or in which the tumor cells form recognizable glandular structures. The term "sarcoma" is art recognized and refers to malignant tumors of mesenchymal derivation.

In a preferred embodiment of the invention said cancer is a carcinoma. Preferably said carcinoma is prostate carcinoma.

The vaccine compositions of the invention can be administered by any conventional route, including injection, intranasal spray by inhalation of for example an aerosol or nasal drops. The administration may be, for example, intravenous, intraperitoneal, intramuscular, intracavity, subcutaneous, or intradermal. The vaccine compositions of the invention are administered in effective amounts. An "effective amount" is that amount of a vaccine composition that alone or together with further doses, produces the desired immune response.

The amounts of vaccine will depend, of course, on the individual patient parameters including age, physical condition, size and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation. It is generally preferred that a maximum dose of the individual components or combinations thereof be used sufficient to provoke immunity; that is, the highest safe dose according to sound medical judgment. It will be understood by those of ordinary skill in the art, however, that a patient may insist upon a lower dose or tolerable dose for medical reasons, psychological reasons or for virtually any other reasons.

The doses of vaccine administered to a subject can be chosen in accordance with different parameters, in particular in accordance with the mode of administration used and the state of the subject. In the event that a response in a subject is insufficient at the initial doses applied, higher doses (or effectively higher doses by a different, more localized delivery route) may be employed to the extent that patient tolerance permits.

In general, doses of vaccine are formulated and administered in effective immunizing doses according to any standard procedure in the art. Other protocols for the administration of the vaccine compositions will be known to one of ordinary skill in the art, in which the dose amount, schedule of injections, sites of injections, mode of administration and the like vary from the foregoing. Administration of the vaccine compositions to mammals other than humans, (e.g. for testing purposes or veterinary therapeutic purposes), is carried out under substantially the same conditions as described above. A subject, as used herein, is a mammal, preferably a human, and including a non-human primate, cow, horse, pig, sheep or goat.

In a preferred embodiment of the invention there is provided a vaccine composition according to the invention that includes at least one additional anti-cancer agent. Preferably said ant-cancer agent is a chemotherapeutic agent.

According to an aspect of the invention there is provided a diagnostic or prognostic method for the detection of cancer cells isolated from a subject comprising determining the expression of NETO-2, wherein over-expression of said gene is indicative of cancer or a predisposition to cancer in said subject.

In a preferred method of the invention said method comprises:
i) providing an isolated biological sample to be tested;
ii) forming a preparation comprising said sample and an oligonucleotide primer pair adapted to anneal to a nucleic acid molecule comprising a nucleic acid sequence as represented in Figure 1a; a thermostable DNA polymerase, deoxynucleotide triphosphates and co-factors;
iii) providing polymerase chain reaction conditions sufficient to amplify said nucleic acid molecule;
iv) analysing the amplified products of said polymerase chain reaction for the presence or absence of a nucleic acid molecule comprising a nucleotide sequence derived from Figure 1 a; and optionally
v) comparing the amplified product with a normal matched control.

In an alternative preferred method of the invention said method comprises:
i) providing an isolated biological sample to be tested;
ii) forming a preparation comprising said sample and an antibody or antibodies that specifically binds one or more polypeptide[s] in said sample as represented by the amino acid sequences presented in Figures 1 b to form an antibody/polypeptide complex;
iii) detecting the complex or complexes so formed; and
iv) comparing the expression of said polypeptide[s] with a normal matched control.

According to a further aspect of the invention there is provided a kit comprising oligonucleotide primer pairs adapted to amplify one or more nucleic acid molecules comprising the nucleotide sequences as represented in Figures 1a.

In a preferred embodiment of the invention said kit further includes components required for polymerase chain reaction.

According to a further aspect of the invention there is provided a kit comprising one or more antibodies adapted to bind one or more polypeptides as represented by the amino acid sequences presented in Figures 1b.

In a preferred embodiment of the invention said kit further includes components required for the detection of bound antibody in an immunoassay.

Throughout the description and claims of this specification, the words "comprise" and "contain" and variations of the words, for example "comprising" and "comprises", means "including but not limited to", and is not intended to (and does not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understo to be applicable to any other aspect, embodiment or example described herein unless incompatible therewith.

An embodiment of the invention will now be described by example only and with reference to the following figures:
Figure 1 illustrates the mRNA and amino acid sequence of NETO-2 Nucleotide sequence of NETO-2 mRNA (3653bp) based on the published sequence (Accession ID: NM_018092.3) (A). Full length 525 amino acid sequence of NETO-2 (B) and the 347 amino acid sequence of the extracellular domain (C);

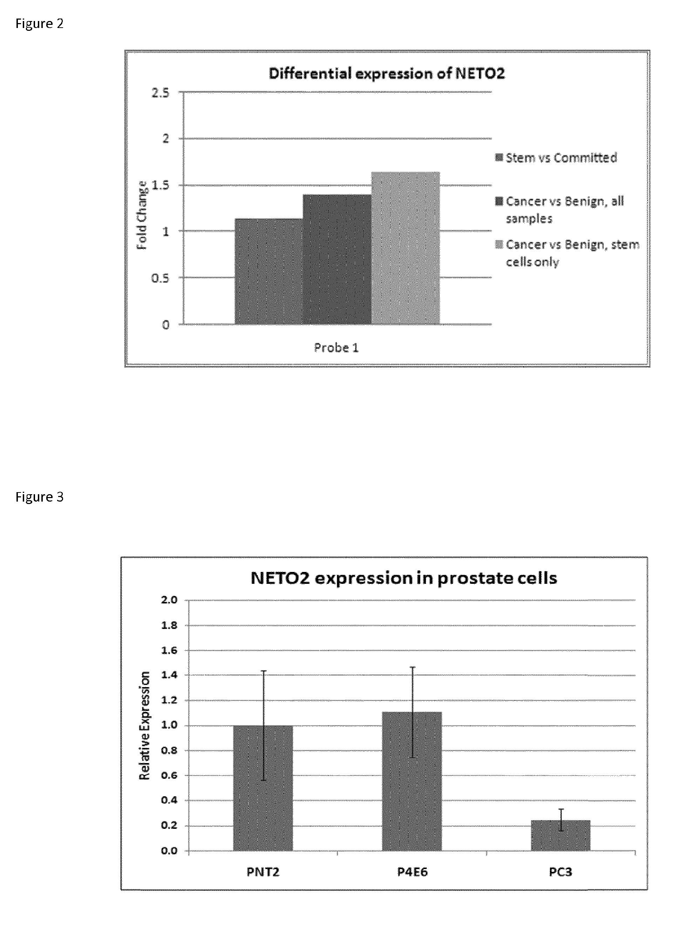

Figure 2 illustrates differential expression of NETO-2 by microarray Differential gene expression for NETO-2 in stem cells versus committed basal in cancer versus benign cells and cancer versus benign stem cells as detected by Affymetrix microarray;
Figure 3 illustrates NETO-2 mRNA expression in prostate cells; NETO-2 mRNA expression level was determined by qRT-PCR in PNT2, P4E6 and PC3 prostate cell lines. Graph shows comparison between cell lines expressed relative to PNT2. All values are the mean ± standard deviation of at least three independent experiments;

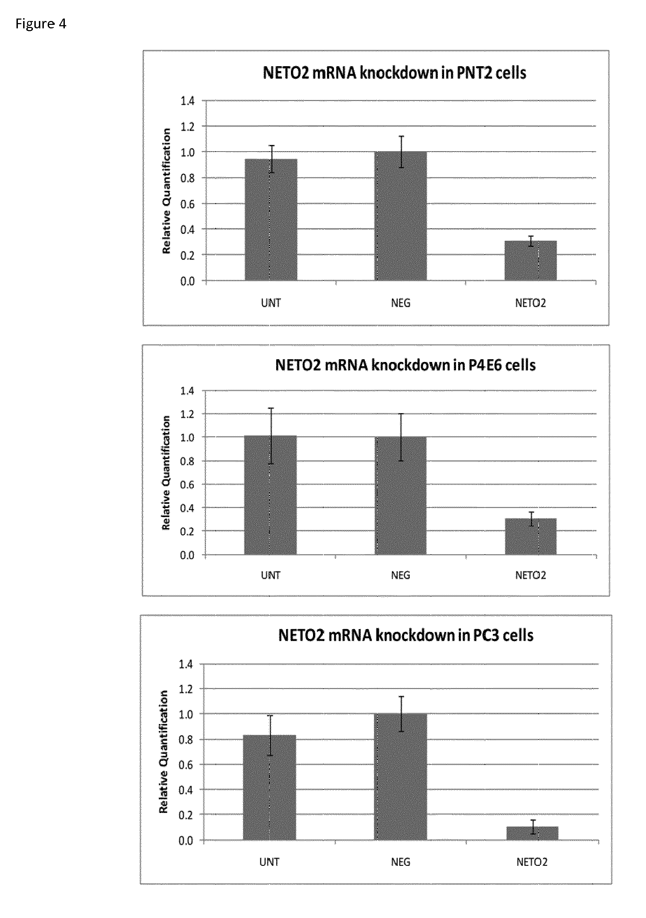

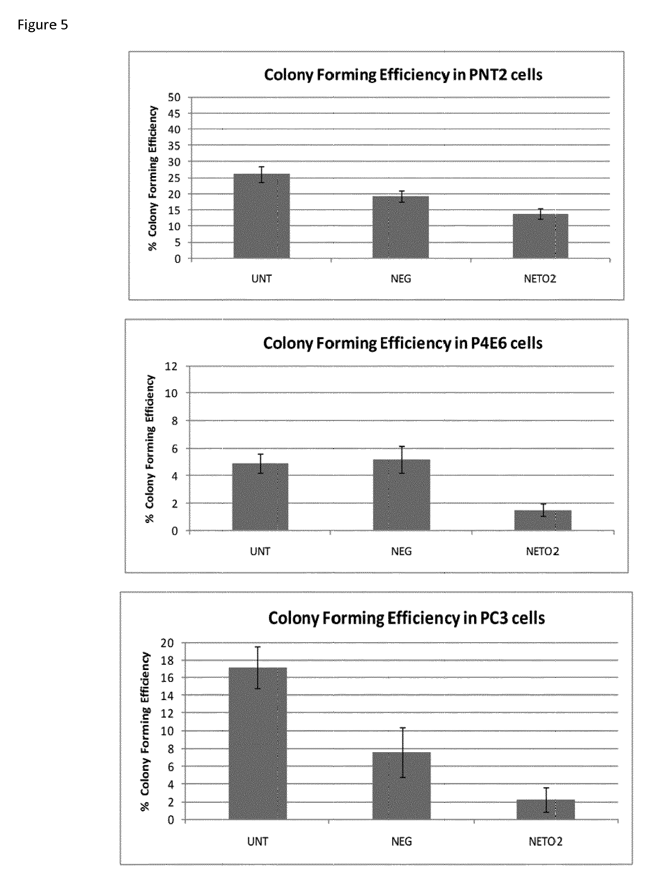

Figure 4 illustrates Inhibition of NETO-2 using siRNA. Effects of target specific siRNA on mRNA levels of NETO-2 in PNT2, P4E6, and PC3. Graphs show comparison of untransfected (UNT) and target-specific siRNA transfected relative to a non specific control siRNA (NEG: negative, scrambled siRNA). The mRNA knockdown data is an average of three independent experiments ± SD, taken from the colony forming efficiency assays shown in Figure 5;
Figure 5 illustrates the effect of NETO-2 siRNA on colony forming efficiency Colony forming efficiency was measured in PNT2 cells and PC3 transfected for 72hrs with media alone (UNT), non-specific siRNA (NEG:negative) or NETO-2 specific siRNA. The graphs show the mean percentage colony forming efficiency from three independent experiments ± SD;

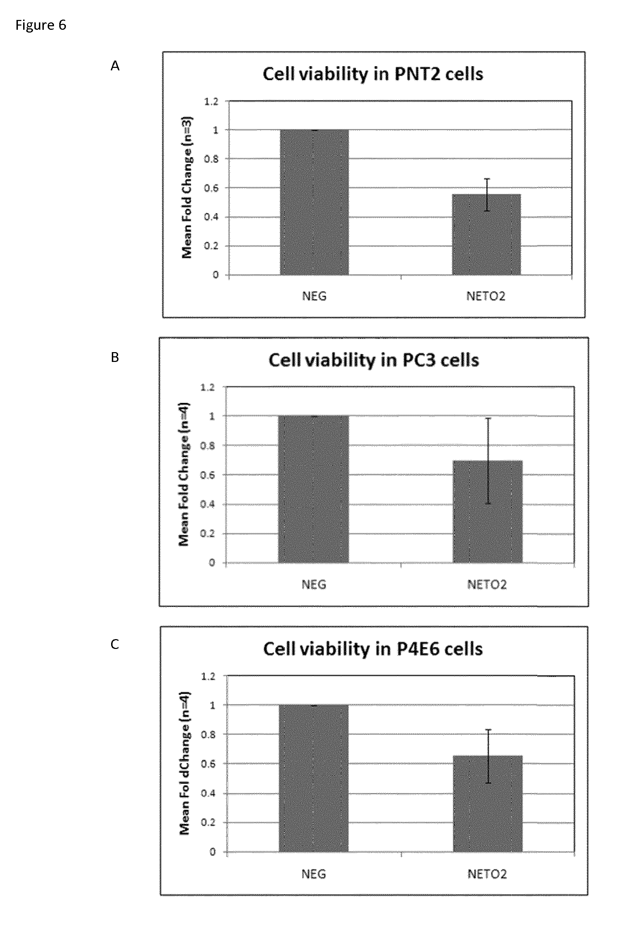

Figure 6 illustrates the effect of NETO-2 siRNA on cell viability (WST assay). Cell viability was measured by WST assay in PNT2 cells, P4E6 cells and PC3 cells transfected for 72hrs with non-specific siRNA (NEG:negative) or NETO-2 specific siRNA. The graphs show the mean fold change from at least three independent experiments ± SD;

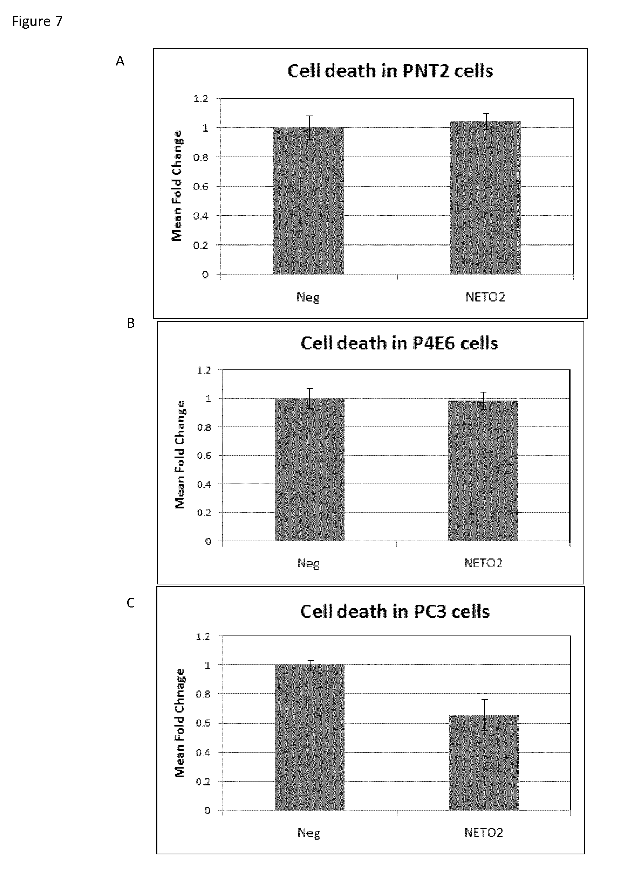

Figure 7 illustrates the effect of NETO-2 siRNA on cell viability (apoptosis assay) The loss of the number of viable cells (i.e. cell death) was measured by a caspase apoptosis assay in PNT2 cells, P4E6 cells and PC3 cells transfected for 72hrs with non-specific siRNA (Neg: negative) or NETO-2 specific siRNA. The graphs show the mean fold change from three independent experiments ± SD; and

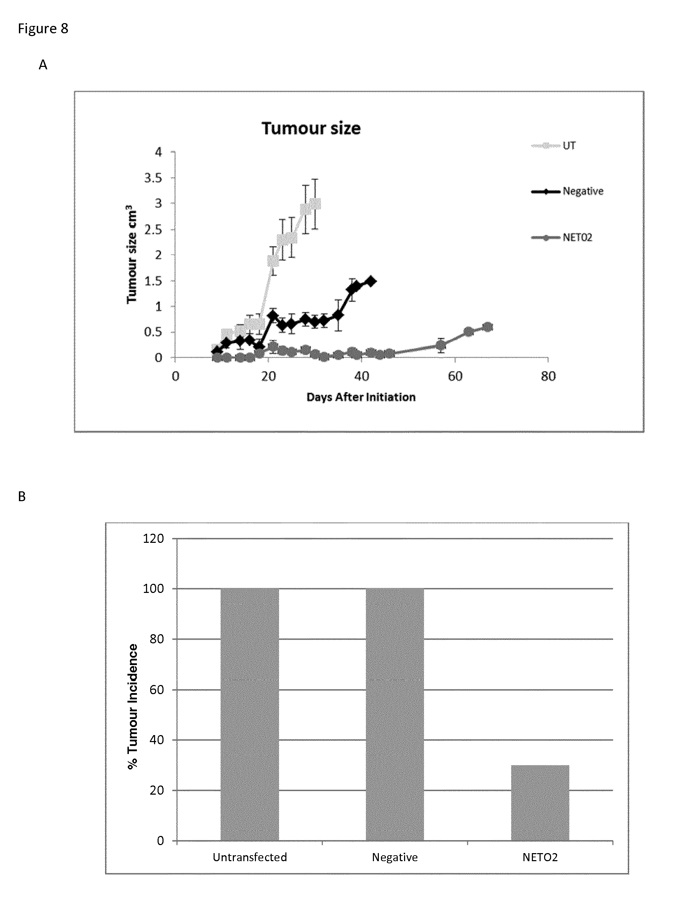

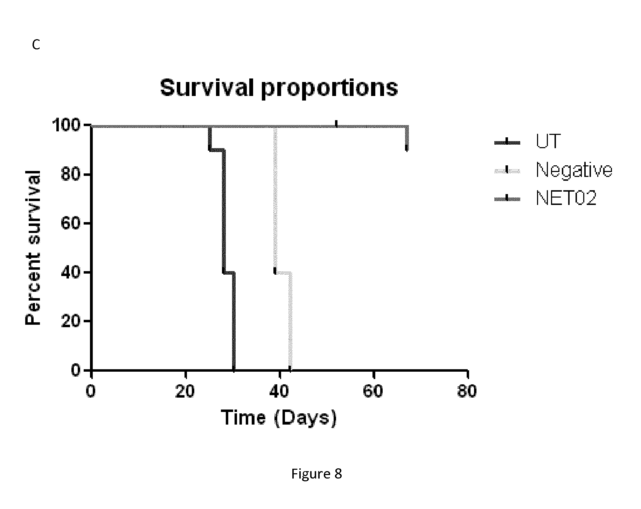

Figure 8 Effect of NETO-2 on *in vivo* tumour formation and survival rates Tumour volume was measured using digital calipers every two days (A) and tumour incidence (B) and survival proportions (C) were calculated. Graphs show the mean tumour volume ± SEM. (n=10 mice per group).

### Materials and Methods

### Generation of NETO-2 Antigen for Antibody Production

Purified His and Fc tagged versions of the ECD (extracellular domain) of NETO2 are prepared for immunisation and screening (∼2mg each). Mice are immunised with selected NETO-2 ECD antigen. Splenic B-lymphocytes are immortalised by fusion to myeloma cells. Hybridomas plated and cloned in one step and screened for affinity, FACS binding and inhibition of cfu and proliferation in relation to cancer/cancer stem cell. Selected clones (up to 4) are expanded and mAbs tested in a mouse tumour xenograft models (PC3 cells and human prostate cancer xenograft. Lead candidate selected for humanisation

### Selection and Characterisation of mAbs to NETO2

Immunisation of mice is carried out with the NETO2 ECD, followed by fusion of splenocytes with a suitable immortalised cell line to form a hybridoma.Anti-NETO2 mAbs produced by the hybridomas undergo primary screening for antigen binding and affinity. Cloning will form part of the fusion and plating process and sequencing will confirm clonality. Four to 6 hybridomas are selected based on optimal in vitro assay criteria and expanded in tissue culture medium and banked in liquid nitrogen as well as other back-up clones. The final lead (and back-up) selection is based on comparative activity in the primary and secondary in vitro screening tests and antitumour efficacy against prostate tumour xenografts in immunocompromised mice with or without cytotoxic drugs.

Q8NC67[23-347], Neuropilin and tolloid-like protein 2, Homo sapien [ECD= bold underlined]

MALERLCSVLKVLLITVLVVEGIAVAQKTQDGQNIGIKHIPATQCGIWVR TSNGGHFASP
NYPDSYPPNKECIYILEAAPRQRIELTFDEHYYIEPSFEC RFDHLEVRDG PFGFSPLIDR
YCGVKSPPLIRSTGRFMWIKFSSDEELEGLGFRAKYSFIPDPDFTYLGGILNPIPDCQFE
LSGADGIVRSSQVEQEEKTKPGQAVDCIWTIKATPKAKIYLRFLDYQMEHSNECKRNF
VAVYDGSSSIENLKAKFCSTVANDVMLKTGIGVIRMWADEGSRLSRFRMLFTSFVEPP
CTSS TFFCHSNMCI NNSLVCNGVQ NCAYPWDENH CKEKKKAGVF EQITKTHGTI
IGITSGIVLV LLIISILVQVKQPRKKVMAC KTAFNKTGFQ EVFDPPHYEL FSLRDKEISA
DLADLSEELDNYQKMRRSSTASRCIHDHHCGSQASSVKQSRTNLSSMELPFRNDFAQP
QPMKTFNSTFKK SSYTFKQGHE CPEQALEDRV MEEIPCEIYV RGREDSAQAS ISIDF

### CANDIDATE IDENTIFICATION AND HUMANISATION

Generation of monoclonal antibodies is well known in the art using established techniques, for example see Antibodies: A Laboratory Manual Ed Harlow, David P Lane 1988 Cold Spring Harbor Laboratory Press, Cold Spring Harbor, New York.
1. Generate mAbs that specifically bind to NETO2
2. Select mAbs on the basis of their ability to inhibit the clonogenic recovery and/or proliferation of PC3 cells and primary cells *in vitro*
3. Evaluate antibody-dependent cellular cytotoxicity (ADCC) and complement mediated cytotoxicity (CDC) enhancement on the activity of anti-NETO2 mAbs in *in vitro* test systems
4. Evaluate apoptosis/cell killing/cell proliferation activity of the anti-NETO2 mAbs in *in vivo* test systems, alone and in combination with known chemotherapeutic agents (e.g Docetaxel)
5. Identify and characterise a lead hybridoma clone for humanisation.

The key steps in the lead identification process are summarised as follows:
1. Mice immunised with selected antigen
2. Splenic B-lymphocytes immortalised by fusion to myeloma cells
3. Hybridomas plated and cloned in one step and screened for affinity, FACS binding and inhibition of cfu and proliferation
4. Best clones (up to 4) expanded and mAbs tested in a mouse tumour xenograft models (PC3 cells and human prostate cancer xenograft)
5. Lead candidate (with back-up) selected for humanisation.
6.

### Establishment of in vitro Screening Tests

The following *in vitro* tests are used to screen and characterise mAb candidates:

### Primary Screen

1. Human NETO2 antigen ELISA

### Secondary Screens

2. NHP NETO2 and human NETO1 binding by ELISA
3. Surface plasmon resonance binding (BIAcore^{®}; NETO2 binding kinetics and epitope mapping)
4. Cell surface binding by fluorescence-activated cell sorting (FACS)
5. Antibody dependent cellular cytotoxicity (ADCC) and/or complement dependent cytotoxicity (CDC), as appropriate
6. Cell fate 1. The effect of the mAb on colony forming (cfu) efficiency of PC3 and primary cells.
7. Cell fate 2. The effects of the mAb candidates on cell fate *in vitro* will be determined in prostate cancer cell lines (PC3) by water-soluble tetrazolium salt (WST) assay and by assay for apoptosis.
8. Cell fate 3. The effects of the mAb candidates on cell fate in prostate cancer stem cells *in vitro* will be measured by apoptosis assays based on flow cytometry using stem cell markers (e.g. CD133) in conjunction with apoptosis markers such as caspases. This will allow the measurement of apoptosis in stem and non-stem cell populations simultaneously.

### Establishment of in vivo Screening Tests

In addition to human xenograft models, we routinely use several different models involving the implantation into mice of prostate cancer cells grown in culture, including PC3 cells. The PC3 cell line was established from a bone metastasis of prostate cancer, it expresses NETO2 and is tumourigenic in mice. We have shown that inhibition of NETO2 by siRNA in these cells *in vitro* leads to a loss of colony forming potential. We have also shown that PC3 cells transfected with anti-NETO2 siRNA are incapable of initiating tumours in mice. Thus we believe this makes the model a suitable preliminary vehicle for *in vivo* analysis of the effects of mAb candidates prior to moving into the primary xenograft models.

### Proposed experiments using the PC3 cell model

Two types of experiment to show the effect of mAbs against NETO2 in an animal model
- Inject PC3 cells subcutaneously into athymic nude mice, and inject the mAb at t=0 and monitor for growth of tumour relative to a control group treated with a non-specific mAb. Five different doses of antibody will be used, and animals will be sacrificed when tumours reach 1.5cm (approx t=6 weeks). A variant of this experiment will be to pre-treat the PC3 cells with the mAb before their injection into the mice and monitor for tumour take relative to the control group.
- Generate established tumours (approximately 2 weeks after injection with PC3 cells) and then to treat with different doses of the mAb as above, monitoring for regression of the tumour or a slower rate of growth relative to the control group.

### Xenografts

We routinely engraft tumour tissue biopsies from patients undergoing radical prostatectomy for prostate cancer. As the biopsies are small (∼2mm) we cannot sort for rare cell populations directly from these samples, and tissue is therefore grafted into immunocompromised mice - either under the kidney capsule or subcutaneously. We have a colony of Rag-2 (-/-) gamma C (-/-) male mice for this purpose because the gamma C knockout renders the mice more susceptible to human tumour engraftment [see also WO2012/101904 which is incorporated by reference in its entirety. This first step also has the advantage of allowing us to select for malignant epithelium relative to normal. The mAbs will be evaluated for tumour response as single agents and in combination with (e.g.) Docetaxel to assess synergy and to measure the effects on time to relapse. Tumours are initiated by grafting selected cell phenotypes orthotopically into the prostate or under the kidney capsule and mice are then randomly assigned to treatment groups (including a placebo group). Treatment is either initiated on the day of grafting or once tumours have become established.

End points are reached once tumours reach 1.5cm and include any observed adverse effects from each therapy. Tumour response will be determined by measurement of tumours when the mice are killed, as well as examination for metastatic spread. The tumours will be retrieved, measured and the fate of the stem cell population determined using our proprietary assays.

These tests will be used to confirm the activity of lead candidates and will also form part of the Non-Clinical Pharmacology package for regulatory submission for the development candidate. Based on positive results of cell fate experiments in PC3 cells *in vitro,* testing of the candidates in the PC3 animal model will be carried out. Testing of the candidates in primary human cells *in vitro* will be performed in parallel with this first round of *in vivo* studies, and will be completed prior to studying the effects *in vivo* with primary human tumour xenografts.

### Selection and Characterisation of mAbs to NETO2

Immunisation of mice is carried out with the NETO2 ECD, followed by fusion of splenocytes with a suitable immortalised cell line to form a hybridoma. Anti-NETO2 mAbs produced by the hybridomas undergo primary screening for antigen binding and affinity. Cloning forms part of the fusion and plating process and sequencing will confirm clonality.

Four to 6 hybridomas are selected based on optimal *in vitro* assay criteria and expanded in tissue culture medium and banked in liquid nitrogen as well as other back-up clones.

### Cell Culture

Prostate cell lines were maintained under standard culture conditions in a humidified incubator at 37°C in 5%CO₂. PNT2 cells were maintained in RPMI 1640 media (Invitrogen, Paisley, UK) with the addition of 10% foetal calf serum (FCS; PAA Laboratories Ltd. Yeovil, UK) and 1% L-Glutamine (Invitrogen, Paisley, UK). PC3 cells were maintained in HAMS F12 (Invitrogen, Paisley, UK) supplemented with 7% foetal calf serum and 1% L-Glutamine and P4E6 cells were grown in keratinocyte serum free medium (Invitrogen, Paisley, UK) with bovine pituitary extract (BPE), epidermal growth factor (EGF), 2% FCS and 1% L-Glutamine.

### qRT-PCR

Reverse transcription was carried out on 50-500ng of fractionated cell RNA to generate cDNA. Real Time PCR was carried out using the Taqman gene expression system (Applied Biosystems, Warrington, UK) according to the manufacturer's protocol with the exception that a reduced total reaction volume of 10µl was used. All reactions were carried out in triplicate in 96-well PCR plates on an ABI Prism 7300 sequence detection system (Applied Biosystems). Standard thermal cycling conditions included a hot start of 2 minutes at 50°C, 10 minutes at 95°C, followed by 40 cycles of: 95°C 15 s, 60°C for 1 minute. Data analysis was carried out using ABI SDS software and Microsoft Excel. 18S was used as endogenous control gene and for normalizing all expression values. For the measurement of RNA knockdown by siRNA differential RNA expression in response to siRNA was calculated by the ΔΔCt method (according to manufacturer, Applied Biosystems).

### Transfection of prostate epithelial cells with siRNA

PNT2, P4E6 and PC3 cells were seeded at 5*10⁴ cells per well of a 24 well plate and incubated overnight prior to transfection. PNT2 and PC3 cells were transfected with Nanofectin (PAA Laboratories Ltd. Yeovil, UK) according to the manufacturer's protocols. P4E6 cells were transfected with Oligofectamine (Invitrogen, Paisley, UK) according to the manufacturer's protocols. In all cases cells were incubated for 72 hours before being assayed for RNA knockdown by qRT-PCR and clonogenicity.

### Colony forming assays in prostate epithelial cells

After treatment with siRNA for 72 hours, cells were plated at 200 cells/well on 24-well plates. Medium was changed every 2-3 days and colony formation was monitored throughout. The endpoint was determined based on the observed proliferation of the negative siRNA control cells (∼7 days for PNT2 and PC3 cells, ∼10days for P4E6 cells). Colony forming efficiency (CFE) was calculated as the number of colonies >32 cells divided by the number of cells initially plated x 100.

### Cell proliferation (WST) assays in prostate epithelial cells

After treatment with siRNA for 72 hrs, cell proliferation was measured using a WST assay. Briefly, cells were treated for 72 hrs with siRNA in triplicate in standard tissue culture media. The media was removed and replaced with a 1:10 dilution of WST-1 reagent in tissue culture media according to the manufacturer's instructions (Roche, Burgess Hill, UK). Cells were subsequently incubated for four hours at 37°C. The absorbance was read at 450nm on a Fluostar Optima plate reader (BMG Labtech). Results are expressed as relative absorbance with respect to cells transfected with non-specific siRNA after background substraction.

### Apoptosis assays in prostate epithelial cells

After treatment with siRNA for 72 hrs, cells were imaged and then cell death was investigated using an apoptosis assay. Cells were washed in MACS buffer (2mM EDTA, 0.5% FCS, PBS) and incubated with CD133-APC antibody for 10 minutes on a circular mixer in the fridge (clone 293C3, Miltenyi Biotec, Bergisch Gladbach, Germany). The cells were rinsed with MACS buffer and incubated with a fluoroisothiocyanate (FITC) conjugate of the cell-permeable caspase inhibitor VAD-FMK (In Situ Caspace Assay, Promega, Southampton, UK) for 20 minutes at 37°C on a rotating mixer. Finally, cells were washed and resuspended in PBS buffer (0.01 M phosphate buffer, 0.0027 M potassium chloride and 0.137 M sodium chloride, pH 7.4) containing DAPI at 1:10,000 concentration for 10 minutes prior to analysis by flow cytometry. Data was collected using a DakoCytomation CyAn ADP instrument (Dako UK Ltd, Cambridgeshire, UK). FACS results were analysed with Summit Software, v4.3 (Dako UK Ltd, Cambridgeshire, UK).

### In vivo Animal Model

PC3 cells were plated at 4x10⁶ per 150cm³ tissue culture flasks and left overnight to adhere. Cells were treated with either NETO2 siRNA, a scrambled control siRNA, or were untransfected and left for 72 hours. Cells were trypsinised and washed, then resuspended in media and counted. Cells were centrifuged and re-suspended at 1x10⁶ cells per 100µl matrigel (BD Matrigel™ Basement Membrane Matrix). Cells were administered subcutaneously into the rear flank of BALB/c Nude under anaesthetic, with ten mice per treatment group. Formation of a bulla indicated satisfactory injection. Thereafter, tumours were measured every two days in terms of length (L), width (W), and height (H) and their volumes calculated according to the formula LxWxHx0.5236. Animals were culled when the tumour reached a size of no more than 1.5cm, or if the animal showed any signs of distress.

### Example 1

### Identification of NETO-2 as a target

The generation of a cancer stem cell gene expression signature using whole genome microarray analysis has been reported previously (Birnie et al, 2008). When gene expression profiles from stem cells and committed basal cells isolated from primary cultures of benign and malignant prostate epithelial cells were compared NETO2 showed an increase in gene expression in stem cells relative to committed basal cells (1.14 fold) (Figure 2).NETO2 was also upregulated in cancer versus benign samples (all cells: 1.39) as well as cancer stem cells relative to benign stem cells (1.64 fold) (Figure 2).

### Example 2

### Expression of NETO-2 in prostate cells

NETO-2 expression was measured in cell lines representing benign prostate epithelium (PNT2), early stage prostate cancer (P4E6) and advanced metastatic prostate cancer

(PC3). Analysis of NETO-2 expression by qRT-PCR showed that NETO-2 is decreased (0.25 fold) (Figure 3). Similar levels of mRNA expression of NETO-2 were observed in P4E6 cells relative to PNT2 cells.

### Example 3

### Inhibition of NETO2 expression using siRNA

Having demonstrated that NETO-2 is expressed in prostate cells, siRNA was used to inhibit the expression in order to investigate the effects on cell fate. Transfection of a NETO-2 specific siRNA reduced NETO-2 mRNA expression by an average of 69% (n=3) in PNT2 cells (Figure 4), by an average of 69% (n=3) in P4E6 cells, and by an average of 89% (n=3) in PC3 cells, relative to a non-specific control siRNA.

### Example 4

### Effect of NETO-2 inhibition on clonogenicity of prostate cells

Clonogenic recovery assays were carried out to determine the ability of the prostate cells treated with NETO-2 siRNA (Figure 5) to form colonies. Results are presented as percent colony forming efficiency (CFE) calculated as follows: (No. of colonies ≥32 cells/no. of cells plated) X 100. Treatment with NETO-2 siRNA showed a small but significant decrease in CFE of 28% (p<0.001) in PNT2 cells. Treatment of P4E6 cells with NETO2 siRNA caused a significant decrease in CFE of 71% (p<0.001). Treatment of PC3 cells with NETO-2 siRNA for 72 hrs resulted in a significant decrease in CFE of 70%, respectively (p<0.05) (Figure 5).

### Example 5

### Effect of NETO-2 inhibition on viability of prostate cells as measured by a WST assay

The effect of NETO-2 inhibition on cell viability was determined using a WST assay. This assay is based on the cleavage of a tetrazolium salt that is added to the culture medium and is irreversibly cleaved by metabolically active cells, releasing a product that can measured on a UV-Vis spectrophotometer. After 72 hrs transfection with non-specific siRNA (NEG) or siRNA specific for NETO-2 of primary prostate epithelial cells, the cell viability was determined. Inhibition of NETO-2 mRNA expression showed decreased cell viability for all three cell lines (loss of cell viability: 45%, 35%, and 29% for PNT2, P4E6 and PC3 cells, respectively, Figure 6). The decrease in cell viability is statistically significant for PNT2 and P4E6 (p<0.009) but not for PC3 (p=0.08).

### Example 6

### Effect of NETO-2 inhibition on viability of prostate cells as measured by an apoptosis assay

The effect of NETO-2 inhibition on cell viability was also determined using a FACS based apoptosis assay. Cell death was monitored by determining the expression of caspase proteins, which are involved in the apoptosis cell signalling pathway. In addition to the caspase inhibitor, DAPI uptake was used as an indicator of compromised integrity of the plasma membrane which is a feature of necrosis. NETO-2 specific siRNA treatment did not show a change in cell death for PNT2 or P4E6 cells (Figure 7). However, there was an increase in cell death in PC3 cells after NETO2 siRNA knockdown (34%, p<0.01)

### Example 7

### Effect of NETO2 on in vivo tumour formation and survival rates

The effect of NETO-2 inhibition on the ability to form tumours *in vivo* was determined by siRNA pre-treatment of PC3 cells, which were then injected into BALB/c Nude mice. Tumour growth was monitored every two days (Figure 8). It was shown that mice given untransfected PC3 cells formed large tumours rapidly as expected, with 100% of mice in the group forming tumours. All mice in this group had to be culled by day 30 post initiation, having reached maximum tumour size allowed. Mice injected with PC3 cells treated with scrambled control siRNA formed tumours in 100% of the mice. These tumours were smaller and took longer to form than the untransfected group, suggesting that siRNA treatment may be affecting tumour growth. All mice in this group were culled by day 42 post initiation. In contrast, pre-treatment of PC3 cells with NETO2 siRNA caused a significant decrease in the size and formation of tumours, with only 30% of mice forming small tumours, and only one mouse being culled at day 67 post initiation, having reached the maximum tumour size permissible. NETO2 siRNA pre-treatment of PC3 cells caused a significant increase in survival proportion compared to both untransfected and scrambled siRNA pre-treated cells, as shown by Kaplan-Meier survival curves. Median survival for untransfected, scrambled siRNA and NETO2 were 28 days, 39 days and undefined respectively.

## Claims

1. A pharmaceutical composition comprising an agent that inhibits tumour initiation wherein the agent comprises an antibody, or active binding fragment thereof, that binds to the extracellular domain of NETO 2 and optionally including a pharmaceutically acceptable carrier.

2. The pharmaceutical composition according to claim 1, wherein said antibody is selected from the group consisting of: a polyclonal antibody, a monoclonal antibody, a chimeric antibody or a humanized or human antibody.

3. The pharmaceutical composition according to claim 1, wherein said active binding fragment is selected from the group consisting of: a single chain antibody fragment, Fab fragment, Fab₂ fragment, F(ab')₂ fragment, Fv fragment, Fc fragment, or Fd fragment.

4. The pharmaceutical composition according to any one of claims 1 to 3, wherein said extracellular domain of NETO2 comprises or consists essentially of the amino acid sequence set forth in Figure 1C.

5. A pharmaceutical composition according to any one of claims 1 to 4 for use in the treatment of cancer.

6. The pharmaceutical composition according to claim 5 wherein said cancer is a carcinoma, preferably prostate carcinoma.

7. A vaccine composition comprising a polypeptide selected from the group consisting of:
i) a polypeptide encoded by a nucleotide sequence as represented in Figure 1a, or an antigenic fragment thereof;
ii) a polypeptide encoded by a nucleotide sequence wherein said sequence is degenerate as a result of the genetic code to the nucleotide sequence defined in (i);
iii) a polypeptide or antigenic fragment comprising an amino acid sequence wherein said sequence is modified by addition deletion or substitution of at least one amino acid residue as represented in Figures 1b, wherein said composition optionally includes an adjuvant and/or carrier.

8. The vaccine composition according to claim 7 wherein said antigenic fragment is an extracellular domain of said polypeptide.

9. The vaccine composition according to claim 8 wherein said extracellular domain comprises or consists of the amino acid sequence as represented in Figure 1c.

10. The vaccine composition according to any of claims 7 to 9 for use in the treatment of cancer.

11. The vaccine composition according to claim 10 wherein said cancer is carcinoma, preferably prostate carcinoma.

12. The composition according to any one of claims 1 to 11 wherein said composition includes a further therapeutic agent.

13. A diagnostic or prognostic method for the detection of cancer cells isolated from a subject comprising determining the expression of NETO-2, wherein over-expression of said gene is indicative of cancer or a predisposition to cancer in said subjectwherein said method comprises:
i) providing an isolated biological sample to be tested;
ii) forming a preparation comprising said sample and an antibody or antibodies that specifically binds one or more polypeptide[s] in said sample as represented by the amino acid sequences presented in Figures 1b to form an antibody/polypeptide complex;
iii) detecting the complex or complexes so formed; and
iv) comparing the expression of said polypeptide[s] with a normal matched control.

14. A diagnostic or prognostic method for the detection of cancer cells isolated from a subject comprising determining the expression of NETO-2, wherein over-expression of said gene is indicative of cancer or a predisposition to cancer in said subjectwherein said method comprises:
i) providing an isolated biological sample to be tested;
ii) forming a preparation comprising said sample and an oligonucleotide primer pair adapted to anneal to a nucleic acid molecule comprising a nucleic acid sequence as represented in Figure 1a; a thermostable DNA polymerase, deoxynucleotide triphosphates and co-factors;
iii) providing polymerase chain reaction conditions sufficient to amplify said nucleic acid molecule;
iv) analysing the amplified products of said polymerase chain reaction for the presence or absence of a nucleic acid molecule comprising a nucleotide sequence derived from Figure 1 a; and optionally
v) comparing the amplified product with a normal matched control.

15. A pharmaceutical compositio comprising an agent that inhibits tumour initiation wherein the agent is selected from the group consisting of: an agent comprising one or more antisense oligonucleotide, antisense RNA molecule, siRNA or shRNA wherein said molecule comprises a nucleotide sequence adapted to anneal to a sense nucleotide sequence represented by the sense sequence presented in Figure 1a and optionally further comprises a carrier adapted to deliver the agent to a cell or tissue.


SEQUENCE LISTING

<110>  The University of York

<120>  Compositions Comprising Agents that Inhibit Neuropilin and Tolloid
Like 2

<130>  4109P/wo

<140>  GB1116702.0
<141>  2011-09-28

<160>  55

<170>  PatentIn version 3.5

<210>  1
<211>  3653
<212>  DNA
<213>  Homo sapiens

<400>  1
acgcggcgct cgcgctccct ccttaaatga gcctgggcgc cccgcgcccg ccacttcagt      60

ggatcccgcg ccggggccgc gggcggagct gcctgccggt cccgcgccgc gcgtccgcac     120

tcctcggccc tcgggcggtc gatgggacgg ggcgccgcgg agcaggaggc ggcgcccgtc     180

ggggtgctcg ggccgcgcgg gagcccactg tggggctcgg gcatggcggg ccgcaggacc     240

tgagctctcc tcaggggagc ggggaggcag ctgctggccg gcgatgggga cggagtgggg     300

ccgtcgccgc cgcgccgagc cgtgagcgcc gagccaccgc cgccgctacc tcagcccttc     360

gcgaagcgcc gggcagctcg ggaacatggc cctggagcgg ctctgctcgg tcctcaaagt     420

gttgttaata acagtactgg tagtggaagg gattgccgtg gcccaaaaaa cccaagatgg     480

acaaaatatt ggaatcaagc atattcctgc aacccagtgt ggcatttggg ttcgaaccag     540

caatggaggt cattttgctt cgccaaatta tcctgactca tatccaccaa acaaggagtg     600

tatctacatt ttggaagctg ctccacgtca aagaatagag ttgacctttg atgaacatta     660

ttatatagaa ccatcatttg agtgtcggtt tgatcacttg gaagttcgag atgggccatt     720

tggtttctct cctcttatag atcgttactg tggcgtgaaa agccctccat taattagatc     780

aacagggaga ttcatgtgga ttaagtttag ttctgatgaa gagcttgaag gactgggatt     840

tcgagcaaaa tattcattta ttccagatcc agactttact tacctaggag gtattttaaa     900

tcccattcca gattgtcagt tcgagctctc gggagctgat ggaatagtgc gctctagtca     960

ggtagaacaa gaggagaaaa caaaaccagg ccaagccgtt gattgcatct ggaccattaa    1020

agccactcca aaagctaaga tttatttgag gttcctagat tatcaaatgg agcactcaaa    1080

tgaatgcaag agaaacttcg ttgcagtcta tgatggaagc agttctattg aaaacctgaa    1140

ggccaagttt tgcagcactg tggccaatga tgtaatgctt aaaacaggaa ttggagtgat    1200

tcgaatgtgg gcagatgaag gtagtcggct tagcaggttt cgaatgctct ttacttcctt    1260

tgtggagcct ccctgcacaa gcagcacttt cttttgccat agcaacatgt gcatcaataa 1320

ttctttagtc tgtaatggtg tccaaaattg tgcataccct tgggatgaaa atcattgtaa 1380

agaaaagaaa aaagcaggag tatttgaaca aatcactaag actcatggaa caattattgg 1440

cattacttca gggattgtct tggtccttct cattatttct attttagtac aagtgaaaca 1500

gcctcgaaaa aaggtcatgg cttgcaaaac cgcttttaat aaaaccgggt tccaagaagt 1560

gtttgatcct cctcattatg aactgttttc actaagggac aaagagattt ctgcagacct 1620

ggcagacttg tcggaagaat tggacaacta ccagaagatg cggcgctcct ccaccgcctc 1680

ccgctgcatc cacgaccacc actgtgggtc gcaggcctcc agcgtcaaac aaagcaggac 1740

caacctcagt tccatggaac ttcctttccg aaatgacttt gcacaaccac agccaatgaa 1800

aacatttaat agcaccttca agaaaagtag ttacactttc aaacagggac atgagtgccc 1860

tgagcaggcc ctggaagacc gagtaatgga ggagattccc tgtgaaattt atgtcagggg 1920

gcgagaagat tctgcacaag catccatatc cattgacttc taatcttctg ctaatggtga 1980

tgtgaattct tagggtgtgt acgtacgcag cctccagggc accatactgt ttccagcagc 2040

caaccctttt ctcccatcac aactacgaag accttgattt accgttaacc tattgtatgg 2100

tgatgttttt attctctcag gcagtctata tatgttaaac caatcaagga acttactcta 2160

ttcagtggaa acaataatca tctctattgc ttggtgtcat ttataggaag cactgccagt 2220

taaagagcat tagaagaggt ggttggatgg agccaggctc aggctgcctc ttcgttttag 2280

caacaagaag actgctcttg actgataaca gctctgtcaa tattttgatg ccacaataaa 2340

cttgattttt ctttacattc cttttatttt tcctttctct aaatttaatt tgttttataa 2400

gcctatcgtt ttaccatttc attttcttac ataagtacaa gtggttaatg taccacatac 2460

ttcagtatag gcatttgttc ttgagtgtgt caaaatacag ctagttactg tgccaattaa 2520

gacccagttg tatttcaccc atctgtttct tcttggctaa tctctgtact tctgcctttt 2580

aattactggg cccttattcc ttattttctg tgagaaataa tagatgatat gatttattac 2640

ctttcaatta tatttttctc agttatacta gaaaatttca taatcctggg atatatgtac 2700

cattgtcagc tatgactaaa aatttgaaaa agataaaaat ttctagcaag cctttgaagt 2760

ttaccaagta tagtcacatt cagtgacagc ccattcattc cagtaaagaa tcatttcatt 2820

cactttggga gaggcctata attacattta tttgcaatgt ttctcttcgc tagattgtta 2880

catagctccc attctgttgg ttttgcttac agcatatggt aaccaaggtt agatgccagt 2940

taaaattcct tagaaattgg atgagccttg agattgcttc ttaactggga catgacattt 3000

ttctagctct tatcaagaat aacaacttcc actttttttt aaactgcact tttgactttt 3060

tttatggtat aaaaacaata atttataaac ataaaagctc attgtgtttt ttagactttt 3120

gatattattt gatactgtac aaactttatt aaatcaagat gaaagaccta caggacagat     3180

tcctttcagt gttcacatca gtggctttgt atgcaaatat gctgtgttgg acctggacgc     3240

tataacttat tgtaaagacc ttggaaatgt ggacataagc tctttctttc cttttgttac     3300

tgtatttagt ttgtgataaa tttttcactg tgtgatattt atgctctaaa tcactacaca     3360

aatcccatat taaaatatac attgtacctg accctttaat catgttattt atgccaccaa     3420

ggttgtggat cttaaggtat gtatggaaag gaactcattt atcaaattgt aagtaataca     3480

gacatgccat ttaaaagagg taaattcttg ttttctatat tttgttagta aattctcaat     3540

gaaataagtt gaagtttcac tggatttcat taacttttaa atattacata tatgtgtttt     3600

ctcagattag tgaaaattgt gaccttaaat ttaatacaca tatactgcct cag            3653

<210>  2
<211>  525
<212>  PRT
<213>  Homo sapiens

<400>  2

Met Ala Leu Glu Arg Leu Cys Ser Val Leu Lys Val Leu Leu Ile Thr
1               5                   10                  15

Val Leu Val Val Glu Gly Ile Ala Val Ala Gln Lys Thr Gln Asp Gly
            20                  25                  30

Gln Asn Ile Gly Ile Lys His Ile Pro Ala Thr Gln Cys Gly Ile Trp
        35                  40                  45

Val Arg Thr Ser Asn Gly Gly His Phe Ala Ser Pro Asn Tyr Pro Asp
    50                  55                  60

Ser Tyr Pro Pro Asn Lys Glu Cys Ile Tyr Ile Leu Glu Ala Ala Pro
65                  70                  75                  80

Arg Gln Arg Ile Glu Leu Thr Phe Asp Glu His Tyr Tyr Ile Glu Pro
                85                  90                  95

Ser Phe Glu Cys Arg Phe Asp His Leu Glu Val Arg Asp Gly Pro Phe
            100                 105                 110

Gly Phe Ser Pro Leu Ile Asp Arg Tyr Cys Gly Val Lys Ser Pro Pro
        115                 120                 125

Leu Ile Arg Ser Thr Gly Arg Phe Met Trp Ile Lys Phe Ser Ser Asp
    130                 135                 140

Glu Glu Leu Glu Gly Leu Gly Phe Arg Ala Lys Tyr Ser Phe Ile Pro

145                 150                 155                 160

Asp Pro Asp Phe Thr Tyr Leu Gly Gly Ile Leu Asn Pro Ile Pro Asp
                165                 170                 175

Cys Gln Phe Glu Leu Ser Gly Ala Asp Gly Ile Val Arg Ser Ser Gln
            180                 185                 190

Val Glu Gln Glu Glu Lys Thr Lys Pro Gly Gln Ala Val Asp Cys Ile
        195                 200                 205

Trp Thr Ile Lys Ala Thr Pro Lys Ala Lys Ile Tyr Leu Arg Phe Leu
    210                 215                 220

Asp Tyr Gln Met Glu His Ser Asn Glu Cys Lys Arg Asn Phe Val Ala
225                 230                 235                 240

Val Tyr Asp Gly Ser Ser Ser Ile Glu Asn Leu Lys Ala Lys Phe Cys
                245                 250                 255

Ser Thr Val Ala Asn Asp Val Met Leu Lys Thr Gly Ile Gly Val Ile
            260                 265                 270

Arg Met Trp Ala Asp Glu Gly Ser Arg Leu Ser Arg Phe Arg Met Leu
        275                 280                 285

Phe Thr Ser Phe Val Glu Pro Pro Cys Thr Ser Ser Thr Phe Phe Cys
    290                 295                 300

His Ser Asn Met Cys Ile Asn Asn Ser Leu Val Cys Asn Gly Val Gln
305                 310                 315                 320

Asn Cys Ala Tyr Pro Trp Asp Glu Asn His Cys Lys Glu Lys Lys Lys
                325                 330                 335

Ala Gly Val Phe Glu Gln Ile Thr Lys Thr His Gly Thr Ile Ile Gly
            340                 345                 350

Ile Thr Ser Gly Ile Val Leu Val Leu Leu Ile Ile Ser Ile Leu Val
        355                 360                 365

Gln Val Lys Gln Pro Arg Lys Lys Val Met Ala Cys Lys Thr Ala Phe
    370                 375                 380

Asn Lys Thr Gly Phe Gln Glu Val Phe Asp Pro Pro His Tyr Glu Leu
385                 390                 395                 400

Phe Ser Leu Arg Asp Lys Glu Ile Ser Ala Asp Leu Ala Asp Leu Ser
                405                 410                 415

Glu Glu Leu Asp Asn Tyr Gln Lys Met Arg Arg Ser Ser Thr Ala Ser
            420                 425                 430

Arg Cys Ile His Asp His His Cys Gly Ser Gln Ala Ser Ser Val Lys
        435                 440                 445

Gln Ser Arg Thr Asn Leu Ser Ser Met Glu Leu Pro Phe Arg Asn Asp
    450                 455                 460

Phe Ala Gln Pro Gln Pro Met Lys Thr Phe Asn Ser Thr Phe Lys Lys
465                 470                 475                 480

Ser Ser Tyr Thr Phe Lys Gln Gly His Glu Cys Pro Glu Gln Ala Leu
                485                 490                 495

Glu Asp Arg Val Met Glu Glu Ile Pro Cys Glu Ile Tyr Val Arg Gly
            500                 505                 510

Arg Glu Asp Ser Ala Gln Ala Ser Ile Ser Ile Asp Phe
        515                 520                 525

<210>  3
<211>  325
<212>  PRT
<213>  Homo sapiens

<400>  3

Ile Ala Val Ala Gln Lys Thr Gln Asp Gly Gln Asn Ile Gly Ile Lys
1               5                   10                  15

His Ile Pro Ala Thr Gln Cys Gly Ile Trp Val Arg Thr Ser Asn Gly
            20                  25                  30

Gly His Phe Ala Ser Pro Asn Tyr Pro Asp Ser Tyr Pro Pro Asn Lys
        35                  40                  45

Glu Cys Ile Tyr Ile Leu Glu Ala Ala Pro Arg Gln Arg Ile Glu Leu
    50                  55                  60

Thr Phe Asp Glu His Tyr Tyr Ile Glu Pro Ser Phe Glu Cys Arg Phe
65                  70                  75                  80

Asp His Leu Glu Val Arg Asp Gly Pro Phe Gly Phe Ser Pro Leu Ile
                85                  90                  95

Asp Arg Tyr Cys Gly Val Lys Ser Pro Pro Leu Ile Arg Ser Thr Gly
            100                 105                 110

Arg Phe Met Trp Ile Lys Phe Ser Ser Asp Glu Glu Leu Glu Gly Leu
        115                 120                 125

Gly Phe Arg Ala Lys Tyr Ser Phe Ile Pro Asp Pro Asp Phe Thr Tyr
    130                 135                 140

Leu Gly Gly Ile Leu Asn Pro Ile Pro Asp Cys Gln Phe Glu Leu Ser
145                 150                 155                 160

Gly Ala Asp Gly Ile Val Arg Ser Ser Gln Val Glu Gln Glu Glu Lys
                165                 170                 175

Thr Lys Pro Gly Gln Ala Val Asp Cys Ile Trp Thr Ile Lys Ala Thr
            180                 185                 190

Pro Lys Ala Lys Ile Tyr Leu Arg Phe Leu Asp Tyr Gln Met Glu His
        195                 200                 205

Ser Asn Glu Cys Lys Arg Asn Phe Val Ala Val Tyr Asp Gly Ser Ser
    210                 215                 220

Ser Ile Glu Asn Leu Lys Ala Lys Phe Cys Ser Thr Val Ala Asn Asp
225                 230                 235                 240

Val Met Leu Lys Thr Gly Ile Gly Val Ile Arg Met Trp Ala Asp Glu
                245                 250                 255

Gly Ser Arg Leu Ser Arg Phe Arg Met Leu Phe Thr Ser Phe Val Glu
            260                 265                 270

Pro Pro Cys Thr Ser Ser Thr Phe Phe Cys His Ser Asn Met Cys Ile
        275                 280                 285

Asn Asn Ser Leu Val Cys Asn Gly Val Gln Asn Cys Ala Tyr Pro Trp
    290                 295                 300

Asp Glu Asn His Cys Lys Glu Lys Lys Lys Ala Gly Val Phe Glu Gln
305                 310                 315                 320

Ile Thr Lys Thr His
                325

<210>  4
<211>  19
<212>  DNA

<213>  Artificial Sequence

<220>
<223>  siRNA

<400>  4
cggaagaatt ggacaacta                                                    19


<210>  5
<211>  19
<212>  DNA
<213>  Artificial Sequence

<220>
<223>  siRNA

<400>  5
caaatgaatg caagagaaa                                                    19


<210>  6
<211>  19
<212>  DNA
<213>  Artificial Sequence

<220>
<223>  siRNA

<400>  6
aggtagaaca agaggagaa                                                    19


<210>  7
<211>  19
<212>  DNA
<213>  Artificial Sequence

<220>
<223>  si RNA

<400>  7
gggagattca tgtggatta                                                    19


<210>  8
<211>  19
<212>  DNA
<213>  Artificial Sequence

<220>
<223>  siRNA

<400>  8
ggagattccc tgtgaaatt                                                    19


<210>  9
<211>  19
<212>  DNA
<213>  Artificial Sequence

<220>

<223>  siRNA

<400>  9
ggactgggat ttcgagcaa                                                    19


<210>  10
<211>  19
<212>  DNA
<213>  Artificial Sequence

<220>
<223>  siRNA

<400>  10
ggaagcagtt ctattgaaa                                                    19


<210>  11
<211>  19
<212>  DNA
<213>  Artificial Sequence

<220>
<223>  siRNA

<400>  11
ccaatgatgt aatgcttaa                                                    19


<210>  12
<211>  19
<212>  DNA
<213>  Artificial Sequence

<220>
<223>  siRNA

<400>  12
ccgaaatgac tttgcacaa                                                    19


<210>  13
<211>  19
<212>  DNA
<213>  Artificial Sequence

<220>
<223>  siRNA

<400>  13
tcaaatgaat gcaagagaa                                                    19


<210>  14
<211>  19
<212>  DNA
<213>  Artificial Sequence

<220>
<223>  siRNA

<400>  14

atgaagagct tgaaggact                                                    19

<210>  15
<211>  19
<212>  DNA
<213>  Artificial Sequence

<220>
<223>  siRNA

<400>  15
actaagggac aaagagatt                                                    19

<210>  16
<211>  19
<212>  DNA
<213>  Artificial Sequence

<220>
<223>  siRNA

<400>  16
gaggagattc cctgtgaaa                                                    19

<210>  17
<211>  19
<212>  DNA
<213>  Artificial Sequence

<220>
<223>  siRNA

<400>  17
ggtagaacaa gaggagaaa                                                    19

<210>  18
<211>  19
<212>  DNA
<213>  Artificial Sequence

<220>
<223>  siRNA

<400>  18
agatgaaggt agtcggctt                                                    19

<210>  19
<211>  19
<212>  DNA
<213>  Artificial Sequence

<220>
<223>  siRNA

<400>  19
gaacaagagg agaaaacaa                                                    19

<210>  20
<211>  19
<212>  DNA
<213>  Artificial Sequence

<220>
<223>  siRNA

<400>  20
cactaagact catggaaca                                                    19


<210>  21
<211>  19
<212>  DNA
<213>  Artificial Sequence

<220>
<223>  siRNA

<400>  21
tggcagactt gtcggaaga                                                    19


<210>  22
<211>  19
<212>  DNA
<213>  Artificial Sequence

<220>
<223>  siRNA

<400>  22
caaattatcc tgactcata                                                    19


<210>  23
<211>  19
<212>  DNA
<213>  Artificial Sequence

<220>
<223>  siRNA

<400>  23
caccaaacaa ggagtgtat                                                    19


<210>  24
<211>  19
<212>  DNA
<213>  Artificial Sequence

<220>
<223>  siRNA

<400>  24
ggagattcat gtggattaa                                                    19


<210>  25
<211>  19
<212>  DNA

<213>  Artificial Sequence

<220>
<223>  siRNA

<400>  25
gtgaaaagcc ctccattaa                                                    19


<210>  26
<211>  19
<212>  DNA
<213>  Artificial Sequence

<220>
<223>  siRNA

<400>  26
aattagatca acagggaga                                                    19


<210>  27
<211>  19
<212>  DNA
<213>  Artificial Sequence

<220>
<223>  siRNA

<400>  27
ctcgggagct gatggaata                                                    19


<210>  28
<211>  19
<212>  DNA
<213>  Artificial Sequence

<220>
<223>  siRNA

<400>  28
cattaaagcc actccaaaa                                                    19


<210>  29
<211>  19
<212>  DNA
<213>  Artificial Sequence

<220>
<223>  siRNA

<400>  29
ctccaaaagc taagattta                                                    19


<210>  30
<211>  19
<212>  DNA
<213>  Artificial Sequence

<220>

<223>  siRNA

<400>  30
gatgaaggta gtcggctta                                                    19


<210>  31
<211>  19
<212>  DNA
<213>  Artificial Sequence

<220>
<223>  siRNA

<400>  31
gcaacatgtg catcaataa                                                    19


<210>  32
<211>  19
<212>  DNA
<213>  Artificial Sequence

<220>
<223>  siRNA

<400>  32
ttgaaaacct gaaggccaa                                                    19


<210>  33
<211>  19
<212>  DNA
<213>  Artificial Sequence

<220>
<223>  siRNA

<400>  33
acaaagagat ttctgcaga                                                    19


<210>  34
<211>  19
<212>  DNA
<213>  Artificial Sequence

<220>
<223>  siRNA

<400>  34
aacaggaatt ggagtgatt                                                    19


<210>  35
<211>  19
<212>  DNA
<213>  Artificial Sequence

<220>
<223>  siRNA

<400>  35

aagcaggagt atttgaaca                                                    19

<210>  36
<211>  19
<212>  DNA
<213>  Artificial Sequence

<220>
<223>  siRNA

<400>  36
tcactaaggg acaaagaga                                                    19

<210>  37
<211>  19
<212>  DNA
<213>  Artificial Sequence

<220>
<223>  siRNA

<400>  37
cagacttgtc ggaagaatt                                                    19

<210>  38
<211>  19
<212>  DNA
<213>  Artificial Sequence

<220>
<223>  siRNA

<400>  38
ggacaaaata ttggaatca                                                    19

<210>  39
<211>  19
<212>  DNA
<213>  Artificial Sequence

<220>
<223>  siRNA

<400>  39
cgtgaaaagc cctccatta                                                    19

<210>  40
<211>  19
<212>  DNA
<213>  Artificial Sequence

<220>
<223>  siRNA

<400>  40
gcttgaagga ctgggattt                                                    19

<210>  41
<211>  19
<212>  DNA
<213>  Artificial Sequence

<220>
<223>  siRNA

<400>  41
gaaaacaaaa ccaggccaa                                                    19


<210>  42
<211>  19
<212>  DNA
<213>  Artificial Sequence

<220>
<223>  siRNA

<400>  42
gattgcatct ggaccatta                                                    19


<210>  43
<211>  19
<212>  DNA
<213>  Artificial Sequence

<220>
<223>  siRNA

<400>  43
ccattaaagc cactccaaa                                                    19


<210>  44
<211>  19
<212>  DNA
<213>  Artificial Sequence

<220>
<223>  siRNA

<400>  44
aatgaatgca agagaaact                                                    19


<210>  45
<211>  19
<212>  DNA
<213>  Artificial Sequence

<220>
<223>  siRNA

<400>  45
ggccaatgat gtaatgctt                                                    19


<210>  46
<211>  19
<212>  DNA

<213>  Artificial Sequence

<220>
<223>  siRNA

<400>  46
ggaagctgct ccacgtcaa                                                    19


<210>  47
<211>  19
<212>  DNA
<213>  Artificial Sequence

<220>
<223>  siRNA

<400>  47
atttgaggtt cctagatta                                                    19


<210>  48
<211>  19
<212>  DNA
<213>  Artificial Sequence

<220>
<223>  siRNA

<400>  48
gtgcataccc ttgggatga                                                    19


<210>  49
<211>  19
<212>  DNA
<213>  Artificial Sequence

<220>
<223>  siRNA

<400>  49
agcaggagta tttgaacaa                                                    19


<210>  50
<211>  19
<212>  DNA
<213>  Artificial Sequence

<220>
<223>  siRNA

<400>  50
gaacaaatca ctaagactc                                                    19


<210>  51
<211>  19
<212>  DNA
<213>  Artificial Sequence

<220>

<223>  siRNA

<400>  51
taatagcacc ttcaagaaa                                                    19


<210>  52
<211>  19
<212>  DNA
<213>  Artificial Sequence

<220>
<223>  siRNA

<400>  52
gcacaagcat ccatatcca                                                    19


<210>  53
<211>  19
<212>  DNA
<213>  Artificial Sequence

<220>
<223>  siRNA

<400>  53
gccaaattat cctgactca                                                    19


<210>  54
<211>  21
<212>  DNA
<213>  Artificial Sequence

<220>
<223>  siRNA sense

<400>  54
ggaguauuug aacaaaucat t                                                 21


<210>  55
<211>  21
<212>  DNA
<213>  Artificial Sequence

<220>
<223>  siRNA antisense

<400>  55
ugauuuguuc aaauacucct g                                                 21